**Europäisches Patentamt**

**European Patent Office**

**Office européen des brevets**

(19)

(11) Veröffentlichungsnummer: **0 073 515**
**B1**

(12) **EUROPÄISCHE PATENTSCHRIFT**

(45) Veröffentlichungstag der Patentschrift:
20.03.85

(51) Int. Cl.⁴: **G 01 N 33/542**

(21) Anmeldenummer: **82107974.6**

(22) Anmeldetag: **30.08.82**

(54) **Verfahren zur Enzymimmuno-Bestimmung in homogener Phase.**

(30) Priorität: **31.08.81 DE 3134361**

(43) Veröffentlichungstag der Anmeldung:
**09.03.83 Patentblatt 83/10**

(45) Bekanntmachung des Hinweises auf die Patenterteilung:
**20.03.85 Patentblatt 85/12**

(84) Benannte Vertragsstaaten:
**AT BE CH DE FR GB IT LI LU NL SE**

(56) Entgegenhaltungen:
**US - A - 4 190 496**

**ANNALS OF CLINICAL BIOCHEMISTRY, Band 16, 1979, M.J. O'SULLIVAN et al. "Enzyme Immunoassay: a Review", Seiten 221 bis 239
BIOCHEMICAL AND BIOPHYSICAL RESEARCH COMMUNICATIONS, Band 40, Nr. 3, 1970, F. MELCHERS et al. "Enhanced stability against heat denaturation of E. coli wild type and mutant beta-galactosidase in the presence of specific antibodies", Seiten 570 bis 575**

(73) Patentinhaber: **Boehringer Mannheim GmbH,
Sandhoferstrasse 116, D-6800 Mannheim 31 (DE)**

(72) Erfinder: **Schrenk, Jürgen, Dr., Am Winkel 4,
D-8121 Wilzhofen (DE)**

(74) Vertreter: **Weickmann, Heinrich, Dipl.-Ing. et al,
Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel
Postfach 860820, D-8000 München 86 (DE)**

ACTORUM AG

## Beschreibung

Die Erfindung betrifft ein Verfahren zur Bestimmung von Antigenen oder Haptenen unter Anwendung des Prinzips des Enzym-Immuno-Verfahrens in homogener Phase und ein Reagens zu seiner Durchführung.

Aufgrund der ständig steigenden Zahl von klinisch-chemischen Analysen von Serumbestandteilen und ähnlichen biologischen Materialien gewinnt die Automatisierbarkeit derartiger Methoden ständig an Bedeutung. Nur durch Automatisierung lässt sich die stark steigende Zahl derartiger Bestimmungen bewältigen und dabei auch der erforderliche Arbeitsaufwand pro Bestimmung herabsetzen.

Ein wesentlicher Fortschritt in der klinischen Diagnostik war die Entwicklung von Verfahren zur Bestimmung von Partnern von Immunreaktionen. So wurde durch die Methode der Radio-Immun-Analyse (RIA) eine wesentliche Erhöhung der Empfindlichkeit derartiger Bestimmungen erreicht. Ein Nachteil des RIA, nämlich die Notwendigkeit, mit radioaktiven Substanzen umzugehen, wurde beseitigt durch den Ersatz der radioaktiven Markierung durch eine Enzymmarkierung, so dass sich der sogenannte Enzym-Immuno-Assay (EIA) ergab. Bei diesem Verfahren lässt man eine bestimmte Menge eines enzymmarkierten Partners der Immunreaktion mit dem unmarkierten, in unbekannter Menge vorliegenden, zu bestimmenden Partner um den gemeinsamen Partner der Immunreaktion, also das spezifisch bindende Protein oder die damit bindefähige Substanz, konkurrieren. Bei der sogenannten ELISA-Methode wendet man den Bindungspartner, um den die zu bestimmende Substanz mit der enzymmarkierten Substanz konkurriert, in fester Form an, um die Trennung von freier und gebundener enzymmarkierter Substanz zu erleichtern. Bei der Durchführung dieses heterogenen Verfahrens auf Analysenautomaten ergeben sich jedoch Probleme, die in der Regel Zusatzeinrichtungen erfordern.

Bekannt ist auch die Durchführung des EIA in homogener Phase, indem die enzymatische Aktivität des Antigen- oder Hapten-Enzymkonjugats durch die Antigen-Antikörper-Reaktion beeinflusst wird. Man nimmt an, dass hierbei der Antikörper die Affinität des Enzymsubstrats zur aktiven Stelle des Enzyms entweder durch sterische Hinderung oder durch eine Konformationsänderung des Enzyms oder durch Verhinderung der für die katalytische Aktivität des Enzyms erforderlichen Konformationsänderung herabsetzt. Auch ist ein Fall bekannt, bei dem das Markerenzym durch das daran gekuppelte Hapten inhibiert, durch den entsprechenden Antikörper aber wieder reaktiv wird. Diese Methoden sind jedoch nur sehr beschränkt anwendbar, da bisher nur wenige geeignete Enzym/Hapten-Kombinationen gefunden wurden, bei denen eine entsprechende Hemmung der Enzymaktivität durch einen der Partner der Immunreaktion erfolgt.

Der Erfindung liegt daher die Aufgabe zugrunde, eine neue Analysenmethode zu finden, die sowohl die Vorteile der hohen Spezifität von Immunreaktionen als auch der hohen Nachweisempfindlichkeit von Markierungsenzymen bietet, also allgemein dem EIA-Prinzip folgt, sich auch in homogener Phase durchführen lässt, jedoch nicht die spezifische Hemmung des Enzyms durch das Hapten bzw. Antigen oder dessen Antikörper erfordert.

Die erfindungsgemässe Lösung dieser Aufgabe bedient sich der Beeinflussung der Hitzedenaturierbarkeit von an Haptene oder Antigene gebundenen Markerenzymen durch hapten- oder antigenspezifische Antikörper.

Das erfindungsgemässe Verfahren zur Bestimmung eines Antigens oder Haptens in homogener wässeriger Phase durch Inkubation in Gegenwart von antigen- oder haptenspezifischen Antikörpern und einer bestimmten Menge an enzymmarkiertem Antigen oder Hapten und Messung der Aktivität des Markerenzyms ist daher dadurch gekennzeichnet, dass man nach der Inkubation die Reaktionslösung unter solchen Temperatur- und Zeitbedingungen erhitzt, bei denen das Markerenzym in Abwesenheit des antigen- oder haptenspezifischen Antikörpers zu mindestens 50% inaktiviert wird, und danach die Enzymaktivität misst. Das erfindungsgemässe Verfahren wird als THERESIA bezeichnet (THErmo-RESistance-Immuno-Assay).

Die Erfindung beruht auf der überraschenden Erkenntnis, dass Antikörper, welche nicht gegen das Markerenzym, sondern gegen das Hapten oder Antigen gerichtet sind, die Hitzedenaturierbarkeit des Markerenzyms so beeinflussen, dass hierauf eine quantitative analytische Bestimmung aufgebaut werden kann.

Beim erfindungsgemässen Verfahren wird das Konjugat aus Hapten bzw. Antigen und Enzym in einer bestimmten, d.h. bekannten Menge eingesetzt. Die Menge lässt sich zwar im Prinzip frei wählen, sie sollte jedoch in der Grössenordnung des in Betracht kommenden Mengenbereichs an zu bestimmendem Hapten oder Antigen in der Probelösung liegen, da freies Hapten bzw. Antigen und das enzymmarkierte Derivat davon ja um den Antikörper konkurrieren müssen.

Der haptenspezifische oder antigenspezifische Antikörper wird ebenfalls in einer bestimmten Menge zugegeben, die von der Menge an zugesetztem Enzymkonjugat abhängt und leicht durch Ausprobieren festgestellt werden kann. Die Menge wird dabei so festgelegt, dass sie gerade ausreicht, um die Hitzedenaturierung des Markerenzyms zu verhindern.

Der Hitzeinaktivierungsschritt selbst wird hinsichtlich der anzuwendenden Temperatur und deren Anwendungsdauer so gewählt, dass das Markerenzym zu mindestens 50% inaktiviert wird. Die Hitzedenaturierung folgt bekanntlich den allgemeinen chemischen Gesetzen und bei zunehmender Temperatur sinkt die Dauer der zur Denaturierung erforderlichen Einwirkung. Im Falle der β-Galactosidase lässt sich beispielsweise eine praktische 100%ige Inaktivierung bei 58°C durch 15minütige Einwirkung, bei 62°C durch 5minütige Einwirkung erzielen, jeweils bei gegebenem Puffer- und pH-Wert. Die Bedingungen des Hitzeschritts werden dabei vorzugsweise so gewählt, dass sie gerade ausreichen, um den angestrebten Inaktivierungsgrad zu erzielen. Dabei ist die einfachste Arbeitsweise dann gegeben, wenn eine 100%ige Inaktivierung gerade erreicht wird, das Verfahren lässt sich jedoch auch bei einer Inaktivierung bis herab zu 50% durchführen. Hierdurch wird die Anwendungsbreite erheblich erweitert, da sich schon eine geringfügige Beeinflussung der Hitzeinaktivierung durch den hapten- oder antigenspezifischen Antikörper für das Verfahren ausnutzen lässt.

Als Markerenzym eignen sich prinzipiell die hitzedenaturierbaren und leicht bestimmbaren Enzyme. Bevorzugt werden Markerenzyme, die aus wenigstens zwei Enzymuntereinheiten bestehen, d. h., mehrere Proteinketten aufweisen. Beispiele hierfür sind Glucoseoxidase, Malatdehydrogenase, Acetylcholinesterase, Katalase und $\beta$-Galactosidase. Letztere wird besonders bevorzugt.

Als Hapten können die für die Bestimmung interessierenden, nicht selbst zur Bildung von Antikörpern befähigten physiologischen oder nichtphysiologischen niedermolekularen Verbindungen verwendet werden, deren Bestimmung klinisch relevant ist. Hierunter fallen beispielsweise Hormone, Wirkstoffe, Enzymsubstrate u. dgl. Beispiele für unphysiologische Haptene sind Arzneimittel und ähnliche Substanzen. Für Antigene gilt im Prinzip das gleiche, sie besitzen lediglich die Fähigkeit, selbst die Antikörperbildung hervorzurufen, während im Falle der Haptene hierzu Konjugate zwischen Hapten und immunogenen Substanzen erforderlich sind. Alle diese Definitionen und Verfahren sind dem Fachmann wohlbekannt und bedürfen hier keiner näheren Erläuterung. Als Beispiele für im Rahmen der Erfindung verwendbare Haptene oder Antigene seien die Schilddrüsenhormone, natürliche und synthetische Steroidhormone Digoxin und Digitoxin, Insulin, Thyrotropin, Ferritin, $\alpha_1$-Fetoprotein, carcinoembryonales Antigen, $HB_2$-Antigen und ähnliche angeführt.

Die Antikörperbildung erfolgt in der bekannten Weise durch Immunisierung geeigneter Versuchstiere oder unter Verwendung von Zellkulturen und Gewinnung des Antiserums, welches entweder als solches oder nach Reinigung des Antikörpers verwendet werden kann. In der Regel kann das so gewonnene Antiserum direkt verwendet werden, eine Reinigung des antigenspezifischen oder haptenspezifischen Antikörpers kann jedoch nach den hierfür üblichen Methoden, beispielsweise Ammonsulfatfraktionierung, Immunosorption u. dgl., die einzeln oder kumuliert angewendet werden können, durchgeführt werden. Im Falle des Haptens wird die Immunisierung mit einem Konjugat des Haptens mit einer zur Antikörperbildung geeigneten Substanz durchgeführt. Auch hierfür eignen sich die dem Fachmann bekannten Substanzen. Bevorzugt werden Proteine, wie Serumalbumine verschiedenen Ursprungs, insbesondere Rinderserumalbumin (RSA) und Humanserumalbumin (HSA) sowie Edestin. Wesentlich ist natürlich, dass das Immunogen von dem verwendeten Markerenzym verschieden ist, um sicherzugehen, dass keine Antikörper gegen das Markerenzym bei der Immunisierung gebildet werden.

Die Messung der Aktivität des Markerenzyms erfolgt nach den hierfür bekannten Methoden und bedarf keiner näheren Erläuterung.

Die oben beschriebene Methode beruht, wie bereits erwähnt, auf der überraschenden Stabilisierung des Markerenzyms im Konjugat mit dem Hapten oder Antigen gegen Hitzedenaturierung durch den nicht gegen das Markerenzym, sondern das Hapten oder Antigen gerichteten Antikörper.

Das erfindungsgemässe Verfahren lässt sich jedoch mit geringer Abwandlung auch dann anwenden, wenn der haptenspezifische bzw. antigenspezifische Antikörper eine derartige Unterdrückung der Hitzeinaktivierung nicht zu bewirken vermag. In diesem Falle setzt man zur Inkubation zusätzlich noch enzymspezifische Antikörper zu, welche das Enzym gegen die normale Hitzedenaturierung zu stabilisieren vermögen. Aus „Biochem. Biophys. Res. Comm.", *40* (1970) 570-575 ist es zwar bereits bekannt, dass enzymspezifische Antikörper ihr Enzym gegen Hitzedenaturierung zu stabilisieren vermögen. Es war jedoch nicht vorhersehbar, dass diese Stabilisierung durch hapten- oder antigenspezifische Antikörper wieder aufgehoben wird und daher ebenfalls beim Verfahren der Erfindung verwendbar ist. Beispielsweise wurde gefunden, dass der Digoxinantikörper zwar nicht in der Lage ist, $\beta$-Galactosidase selbst gegen Hitzedenaturierung zu stabilisieren. Er verhindert jedoch die Stabilisierung dieses Enzyms durch den $\beta$-Galactosidaseantikörper.

Bei derjenigen Ausführungsform des erfindungsgemässen Verfahrens, die in Gegenwart des enzymspezifischen Antikörpers durchgeführt wird, wird die Menge an enzymspezifischem Antikörper und an hapten- oder antigenspezifischem Antikörper wie oben näher erläutert festgelegt. In diesem Falle bestimmt man zuerst diejenige Menge an enzymspezifischem Antikörper, die unter festgelegten Temperatur- und Zeitbedingungen die Hitzeinaktivierung des Enzyms verhindert und anschliessend diejenige Menge an hapten- oder antigenspezifischem Antikörper, die diese Stabilisierung wieder aufhebt. In Gegenwart von freiem Hapten oder Antigen, welches mit seinem Antikörper einen Komplex eingeht, wird die Hitzestabilisierung durch den enzymspezifischen Antikörper wieder wirksam, und zwar in dem Masse, in dem sich Hapten- oder Antigen-Antikörper-Komplexe gebildet haben.

Die Herstellung des Enzym-Hapten- bzw. Enzym-Antigen-Konjugats und des Hapten-Immunogen-Konjugats erfolgt nach bekannten Methoden, vorzugsweise unter Verwendung bifunktioneller Brückenbildnerreagenzien. Geeignete Methoden sind beispielsweise beschrieben in „Annals of Chimical Biochemistry", *16* (1979), 221-239.

Das erfindungsgemässe Verfahren weist eine sehr hohe Empfindlichkeit auf und ermöglicht daher die Bestimmung äusserst geringer Mengen an Haptenen und Antigenen, insbesondere bei der Ausführungsform in Gegenwart von enzymspezifischen Antikörpern. So gelingt es in der Probe, Mengen in der Grössenordnung von wenigen Picogramm innerhalb kurzer Zeit zu bestimmen, wobei für die Gesamtheit der Schritte, also Inkubation, Hitzeschritt, Enzymbestimmung nicht mehr als 20 bis 30 min erforderlich sind. Da das Verfahren keine Trennoperationen erfordert, eignet es sich vorzüglich zur Durchführung auf handelsüblichen Analysenautomaten. Als einzige apparative Veränderung ist in der Regel nur der Einbau einer Hitzestrecke erforderlich, falls der Automat eine solche noch nicht aufweist.

Hinsichtlich pH-Wert und verwendeter Puffersubstanz gelten für das erfindungsgemässe Verfahren die durch das Markerenzym vorgegebenen Bedingungen. Dies bedeutet, dass je nach verwendetem Markerenzym ein pH-Wert und eine Puffersubstanz verwendet werden, welche eine ausreichende Aktivität des Enzyms sicherstellen. Diese Bedingungen sind für die in Betracht kommenden Enzyme bekannt.

Ein weiterer Gegenstand der Erfindung ist ein Reagens zur Durchführung des erfindungsgemässen Verfahrens, welches gekennzeichnet ist durch einen Gehalt an einer bestimmten Menge β-Galactosidase-Haptenkonjugat, Hapten-Antikörper, Puffer pH 6,0 bis 8,5, ein System zur Bestimmung der β-Galactosidase-Aktivität und gegebenenfalls eine bestimmte Menge β-Galactosidase-Antikörper. Die Haptenkonjugat-Menge beträgt zweckmässig 100 pmol bis 5 µmol/l, entsprechend 0,01 bis 50 mU/Test; die Antikörper werden vorzugsweise als Vollserum, Konz. bis $10^{-3}$ verdünnt, eingesetzt.

Im Falle dieses bevorzugten Reagens besteht das System zur Bestimmung der Enzymaktivität zweckmässig aus einem Galactosid, welches einen optisch bestimmbaren Substituenten trägt, welcher durch die β-Galactosidase abgespalten wird und leicht optisch bestimmt werden kann. Ein Beispiel für ein derartiges, optisch bestimmbares Substrat ist o-Nitrophenyl-β-D-galactosid (ONPG), welches bevorzugt in einer Konzentration von 1 bis 10 mmol bei 25 bis 42° C eingesetzt wird.

Das erfindungsgemässe Reagens eignet sich insbesondere zur Anwendung auf Analysenautomaten, darunter auch solchen, die nach dem Zentrifugalanalysen-Prinzip arbeiten. Aufgrund der ausserordentlich hohen Empfindlichkeit ist der Reagensaufwand gering, Gleiches gilt für den Arbeitsaufwand.

Die folgenden Beispiele erläutern die Erfindung weiter.

*Beispiel 1*

*Bestimmung von Thyroxin (T₄)*

Puffer: 10 mmol K-Phosphat, pH 7,0
       0,14 mol NaCl
       0,5 mmol MgCl₂
       1 mmol DTT (1,4-Dithiothreitol)
Reaktionsmischung:

10 µl T₄-derivatisierte β-Galactosidase 1 µg/ml (hergestellt durch Umsetzung von Jodacetyl-T₄ mit β-Galactosidase im Molverhältnis 1:2)
10 µl Anti-T₄-Schafsvollserum, $10^{-1}$ verdünnt (Immunogen: T₄-Edestin-Konjugat)
20 µl L-Thyroxin, 1,25 bis 25 ng/l

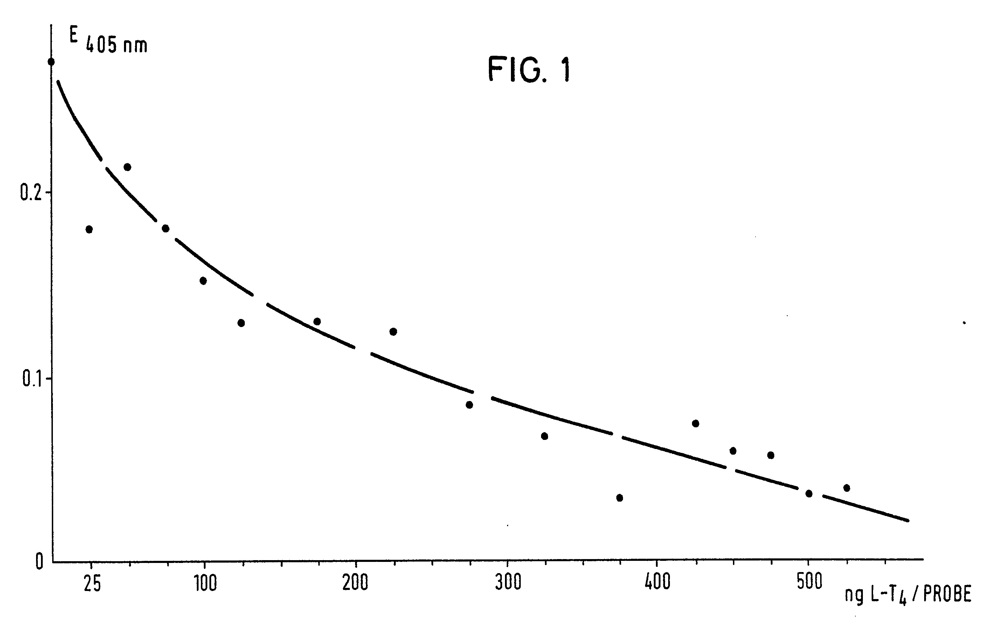

Die obige Mischung wird 30 min bei 37° C inkubiert und dann 5 min auf 62° C erhitzt. Anschliessend werden 70 µl ONPG-Lösung (o-Nitrophenyl-β-D-galactosid; 3,3 mg/ml) zugegeben, 45 min bei 37° C gehalten und dann 1 ml 0,1 mol Na₂CO₃-Lösung zum Abstoppen der Enzymreaktion zugesetzt. Anschliessend wird freigesetztes Nitrophenol bei 405 nm gemessen. Die Nitrophenolmenge ist umgekehrt proportional der Menge an freiem T₄, das mit der derivatisierten β-Galactosidase um den stabilisierenden Antikörper konkurriert. Fig. 1 der beigefügten Zeichnung zeigt in graphischer Darstellung die bei unterschiedlichen Mengen an eingesetztem T₄ erfindungsgemäss erhaltene Eichkurve. In dieser ist auf der Abszisse die eingesetzte T₄-Menge in Nanogramm, auf der Ordinate die Extinktion bei 405 nm angegeben.

*Beispiel 2*

Es wurde wie in Beispiel 1 beschrieben vorgegangen, jedoch wurde anstelle von T₄-derivatisierter β-Galactosidase digoxinderivatisierte β-Galactosidase eingesetzt. Dieses Konjugat wurde hergestellt durch Umsetzung von Digoxinhydroxysuccinimidester mit β-Galactosidase im Molverhältnis 20:1. Die Anti-β-Galactosidase-Serummenge betrug 50 µl, 1:100 verdünnt. Die Anti-Digoxinmenge (Schaf) betrug 50 µl einer 1:5 verdünnten Ammonsulfatsuspension. Für die Immunisierung wurde Digoxin-Edestin-Konjugat als Immunogen verwendet.

Die Hitzedenaturierung erfolgte durch 5minütiges Erhitzen auf 62° C. Die Messung mit ONPG erfolgte 10 min bei 37° C.

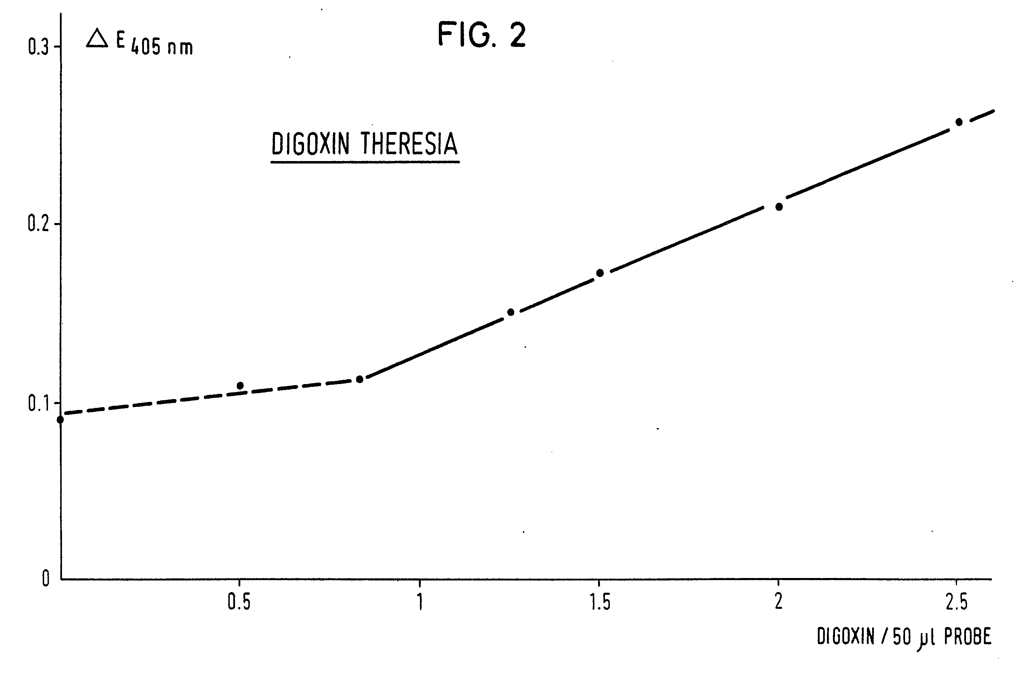

Die mit unterschiedlichen Mengen an Digoxin-Standardlösung erhaltene Eichkurve zeigt Fig. 2 der beigefügten Zeichnung. In der Abszisse ist die Digoxinmenge in Picogramm pro 50 µl Probe, in der Ordinate die Extinktionsdifferenz bei 405 nm angegeben.

**Patentansprüche**

1. Verfahren zur Bestimmung eines Antigens oder Haptens in homogener wässeriger Phase durch Inkubation in Gegenwart von antigen- oder haptenspezifischen Antikörpern und einer bestimmten Menge an enzymmarkiertem Antigen oder Hapten und Messung der Aktivität des Markerenzyms, dadurch gekennzeichnet, dass man nach der Inkubation die Reaktionslösung unter solchen Temperatur- und Zeitbedingungen erhitzt, bei denen das Markerenzym in Abwesenheit des antigen- oder haptenspezifischen Antikörpers zu mindestens 50% inaktiviert wird, und danach die Enzymaktivität misst.

2. Abwandlung des Verfahrens nach Anspruch 1, dadurch gekennzeichnet, dass zur Inkubation zusätzlich enzymspezifische Antikörper zugesetzt werden und die Temperatur- und Zeitbedingungen der Erhitzung so gewählt werden, dass das Markerenzym in Abwesenheit des enzymspezifischen Antikörpers zu mindestens 50% inaktiviert wird.

3. Verfahren nach einem der Ansprüche 1 oder 2, dadurch gekennzeichnet, dass man als Markerenzym ein aus mindestens zwei Untereinheiten bestehendes Enzym verwendet.

4. Verfahren nach Anspruch 3, dadurch gekennzeichnet, dass man β-Galactosidase als Markerenzym verwendet.

5. Reagens zur Durchführung des Verfahrens nach einem der Ansprüche 1 bis 4, gekennzeichnet durch einen Gehalt an einer bestimmten Menge β-Galactosidase-Haptenkonjugat, Hapten-Antikörper, Puffer pH 6,0 bis 8,5, ein System zur Bestimmung der β-Galactosidase-Aktivität und gegebenenfalls eine bestimmte Menge β-Galactosidase-Antikörper.

6. Reagens nach Anspruch 5, dadurch gekennzeichnet, dass die β-Galactosidase-Haptenkonjugat-Menge 100 pmol bis 5 μmol/l beträgt und der bzw. die Antikörper in Form von Vollserum vorliegen.

7. Reagens nach einem der Ansprüche 5 oder 6, dadurch gekennzeichnet, dass das System zur Bestimmung der Enzymaktivität aus einem Galactosid besteht, welches einem durch β-Galactosidase abspaltbaren, optisch bestimmbaren Substituenten trägt.

8. Reagens nach Anspruch 7, dadurch gekennzeichnet, dass es 1 bis 10 mmol o-Nitrophenyl-β-D-galactosid enthält.

## Revendications

1. Procédé pour la détermination d'un antigène ou d'un haptène en phase aqueuse homogène par incubation en présence d'anticorps spécifiques de l'antigène ou de l'haptène et d'une quantité déterminée d'antigène ou d'haptène marqué par une enzyme, et mesure de l'activité de l'enzyme marqueuse, caractérisé en ce que, après l'incubation, on chauffe la solution de réaction dans les conditions de température et de durée dans lesquelles l'enzyme marqueuse, en l'absence de l'anticorps spécifique de l'antigène ou de l'haptène, est inactivée à au moins 50%, puis on mesure l'activité enzymatique.

2. Variante du procédé selon la revendication 1, caractérisée en ce que, pour l'incubation, on ajoute en outre des anticorps spécifiques de l'enzyme et on choisit les conditions de température et les durées du chauffage de manière que l'enzyme marqueuse soit inactivée à au moins 50% en l'absence de l'anticorps spécifique de l'enzyme.

3. Procédé selon l'une des revendications 1 ou 2, caractérisé en ce que l'on utilise en tant qu'enzyme marqueuse une enzyme consistant en au moins deux sous-unités.

4. Procédé selon la revendication 3, caractérisé en ce que l'on utilise en tant qu'enzyme marqueuse la β-galactosidase.

5. Réactif pour la mise en œuvre du procédé selon l'une des revendications 1 à 4, caractérisé en ce qu'il contient une quantité déterminée de produit de conjugaison β-galactosidase/haptène, un anticorps de l'haptène, du tampon pH 6,0 à 8,5, un système pour la détermination de l'activité de la β-galactosidase et, le cas échéant, une quantité déterminée d'anticorps de la β-galactosidase.

6. Réactif selon la revendication 5, caractérisé en ce que la quantité du produit de conjugaison β-galactosidase/haptène est de 100 pmol à 5 μmol/l, et en ce que le ou les anticorps sont à l'état de sérum complet.

7. Réactif selon l'une des revendications 5 ou 6, caractérisé en ce que le sytème pour la détermination de l'activité enzymatique consiste en un galactoside qui porte un substituant clivable par la β-galactosidase et dosable par des méthodes optiques.

8. Réactif selon la revendication 7, caractérisé en ce qu'il contient de 1 à 10 mmol d'o--nitrophényl-β-D-galactoside.

## Claims

1. Process for the determination of an antigen or hapten in homogeneous aqueous phase by incubation in the presence of antigen- or hapten-specific antibodies and of a definite amount of enzyme-marked antigen or hapten and measurement of the activity of the marker enzyme, characterised in that, after incubation, one heats the reaction solution under those temperature and time conditions at which the marker enzyme is inactivated by at least 50%, in the absence of the antigen- or hapten-specific antibody, and thereafter measures the enzyme activity.

2. Modification of the process according to Claim 1, characterised in that, for the incubation, there are additionally added enzyme-specific antibodies, and the temperature and time conditions of the heating are so chosen that the marker enzyme is inactivated by at least 50% in the absence of the enzyme-specific antibody.

3. Process according to one of Claims 1 or 2, characterised in that as marker enzyme one uses an enzyme consisting of at least two sub-units.

4. Process according to Claim 3, characterised in that one uses β-galactosidase as marker enzyme.

5. Reagent for the carrying out of the process according to one of Claims 1 to 4, characterised by a content of a definite amount of β-galactosidase/hapten conjugate, hapten antibody, buffer pH 6.0 to 8.5, a system for the determination of the β-galactosidase activity and optionally a definite amount of β-galactosidase antibody.

6. Reagent according to Claim 5, characterised in that the amount of β-galactosidase/hapten conjugate amounts to 100 pmol to 5 μmol/l and the antibody or antibodies are present in the form of whole serum.

7. Reagent according to one of Claims 5 or 6, characterised in that the system for the determination of the enzyme activity consists of a galactoside which carries an optically determinable substituent which can be split off by β-galactosidase.

8. Reagent according to Claim 7, characterised in that it contains 1 to 10 mmol o-nitrophenyl-β-D-galactoside.

FIG. 1

FIG. 2

$\triangle E_{405\,nm}$

DIGOXIN THERESIA

DIGOXIN / 50 µl PROBE

0 073 515